# EUROPEAN PATENT APPLICATION

(11) **EP 0 943 329 A1**
(43) Date of publication of application: **22.09.1999**
(21) Application number: 99301498.4
(22) Date of filing: 01.03.1999
(51) Int. Cl.: A61K 31/135, A61K 31/445

(54) **Fluoxetine hydrochloride for decreasing hot flashes**

(30) Priority: 02.03.1998 US 76541 P; 21.01.1999 US 116570 P
(71) Applicant: ELI LILLY AND COMPANY, Indianapolis, Indiana 46285 (US)
(72) Inventor: Heath, Hunter, Indianapolis, Indiana 46234 (US); Plouffe, Leo, Jr., Carmel, Indiana 46032 (US)
(74) Representative: Vaughan, Jennifer Ann (GB)

(57) **Abstract**

The present invention includes a method for decreasing hot flashes in a human female by administering fluoxetine to that female. Another aspect of the invention is a method for decreasing hot flashes in a human female undergoing raloxifene administration by administrating fluoxetine to that female. Another aspect of the invention is a pharmaceutical formulation comprising fluoxetine and raloxifene.

## Description

The instant invention relates to the use of fluoxetine hydrochloride for decreasing hot flashes(otherwise known as "hot flushes").

In general, menopause is associated with vasomotor symptoms, manifested by hot flashes, flushing, or night sweats, which are variable in frequency and severity, and may persist for several months or a few years. Approximately 75% of menopausal women will experience flashes during menopause (McKinlay, S., Jeffreys, M., "The Menopausal Syndrome," *J. Prev. Soc. Med.,* 28:108, 1974), with 80% experiencing them for greater than one year and 25 to 50% for greater than 5 years. Judd, H.L., Cleary, R.E., Creasman, W.T., *et al.,* "Estrogen Replacement Therapy," *Obstet. Gynecol.,* 58:267, 1981. For some of these women, the symptoms are disabling. Gambrell, R.D. Jr., "The Menopause: Benefits and Risks of Estrogen-Progestogen Replacement Therapy," *Fertil. Steril.,* 37:457, 1982. The standard therapy for alleviating these symptoms is estrogen replacement therapy (ERT). Many women, unfortunately, are not candidates for ERT therapy because such therapy is medically contraindicated (*e.g.,* estrogen sensitive carcinoma and thromboembolic disease). Furthermore, this therapy, while effective, suffers from poor patient compliance, due to unpleasant side-effects, poor oral absorption, and poor bio-availability of the natural estrogens 17β-estradiol and estrone.

Non-hormonal alternatives for hot-flashes are extremely limited at present and have been associated with poor response in many patients. The two most widely used non-hormonal therapeutic modalities at present in the United States are transdermal clonidine and Bellargal spacetabs. Neither has gained wide clinical acceptance because of poor effectiveness and side effects.

A recent report has established in a pilot study that 50% of tamoxifen treated menopausal women (survivors of breast cancer) suffering from hot flashes reported a significant improvement of their hot flashes during tamoxifen therapy when sertraline, a selective serotonin reuptake inhibitor (SSRI) of the formula: 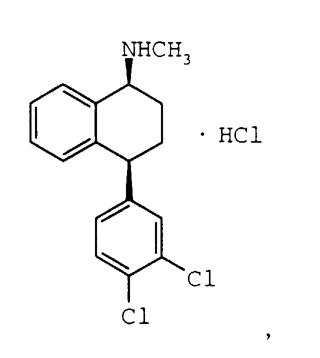 was also administered. Plouffe, *et al.,* "An Open Trial of Sertraline for Menopausal Hot Flushes: Potential Involvement of Serotonin in Vasomotor Instability", *Del. Med. J.,* 69(9):481-2, 1997. The investigators believed that the hot flash mechanism of action may be mediated through serotonin. However, the investigators also noted that "unpublished clinical trials with various serotoninergic agents in menopausal women have failed to show any relief from hot flushes." *Id.* at 481. Thus, although the hot flash mechanism may indeed be mediated through serotonin, it can not be predicted *a priori* whether a pharmaceutical that is classified as a SSRI is effective at decreasing the incidence of hot flashes.

The present invention includes a method for decreasing hot flashes in a human female comprising administering an effective amount of fluoxetine to a human female in need thereof.

Another aspect of the invention is a method for decreasing hot flashes in a human female undergoing raloxifene administration comprising administrating fluoxetine to a human female in need thereof an effective amount of fluoxetine.

Another aspect of the invention is a pharmaceutical formulation comprising fluoxetine and raloxifene.

Further aspects of the present invention include a use of fluoxetine for the manufacture of a medicament for decreasing hot flashes in a human female and a use of fluoxetine for the manufacture of a medicament for decreasing hot flashes in a human female undergoing raloxifene administration.

Fluoxetine hydrochloride (fluoxetine) is a well known SSRI. See, *e.g.*, U.S. Patent No. 4,590,213. Relative to clonidine and bellargal, fluoxetine has a favorable side effect profile. Fluoxetine has the following structure:

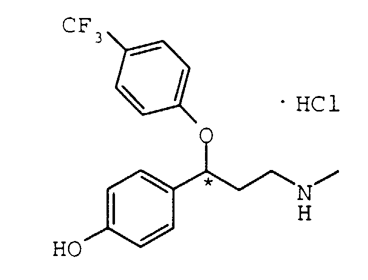

The carbon atom designated above with an asterisk (*) is a chiral center. Thus, fluoxetine is enantiomeric, *i.e.*, has an "R" and "S" enantiomer, both of which are shown below:

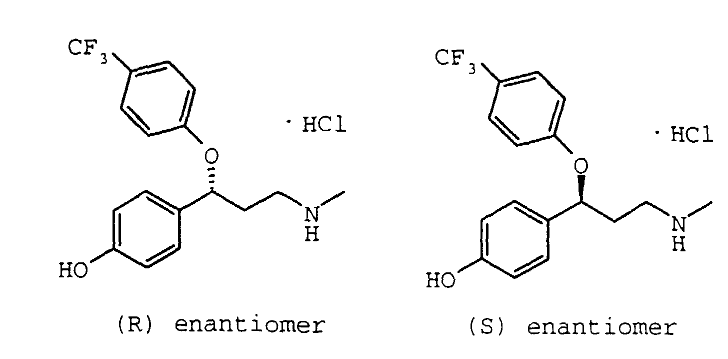

The present invention envisions the use of a racemic mixture of fluoxetine, the (R)-enantiomer, the (S)-enantiomer, or a mixture enhanced with one enantiomer. An "enhanced" mixture is where one enantiomer makes up between 50% and 99% of the total enantiomeric mixture. A preferred embodiment of the present invention is the use of a racemic mixture or a mixture 90%-99% enhanced with the (R)-enantiomer. Most preferred is the use of substantially pure (R)-enantiomer. As used in the present application, "substantially pure" means that the enantiomeric mixture contains at least 99% by weight (R)-fluoxetine and 1% or less of (S)-fluoxetine.

Raloxifene hydrochloride (raloxifene) is described in US patent No. 4,418,068 and is known to be effective in treating the symptoms of post menopausal syndrome, particularly osteoporosis. Indeed, raloxifene was approved for marketing as a preventative agent for osteoporosis by the U.S. Food and Drug Administration in late 1997. Raloxifene has the following structure: 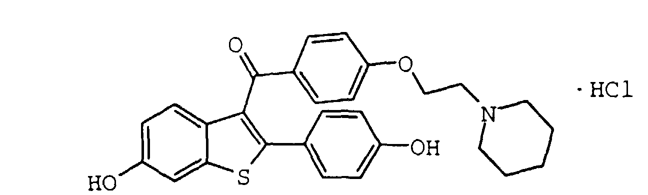 Clinical studies of raloxifene demonstrated a slight increase in the number of women, relative to placebo, who reported incidences of hot flashes during the clinical trial. (24.6% for raloxifene vs. 18.3% for placebo).

Thus, another aspect of this invention, is a method for decreasing hot flashes in a human female undergoing raloxifene administration by administration of fluoxetine.

When "fluoxetine" and/or "raloxifene" are referred to it is understood that such terms refer to fluoxetine hydrochloride (the racemate, individual enantiomers, or mixtures thereof) and raloxifene hydrochloride, respectively, and includes other salts and solvates thereof.

The term "effective amount of fluoxetine" refers to an amount of fluoxetine that is capable of decreasing hot flashes. "Decreasing hot flashes" is defined to include either reducing the occurrences or severity of the hot flashes. The effective amount (per day) of fluoxetine will typically be in the range from about 1 mg to 200 mg per day, usually being in the range from about 5 mg to 80 mg per day, preferably being in the range from about 10 mg to 60 mg per day, and more preferably being in the range from about 15 mg to 40 mg per day.

The term "effective amount of raloxifene" refers to an amount which inhibits bone loss or an amount which is used for any other medical therapeutic reason.
When fluoxetine is administered with raloxifene, the effective amount (per day) of raloxifene will typically be in the range from about 10 mg to 1000 mg per day, usually being in the range from about 10 mg to 100 mg per day, preferably being in the range from about 25 mg to 75 mg per day, more preferably being in the range from about 55 mg to 65 mg per day, and most preferably being 60 mg per day.

The term "effective term" refers to the period of time that a human female suffers from incidences of hot flashes and is usually for greater than 6 months. The effective term should be determined by the caregiver in consult with the caregiver's human female patient.

The term "human female" as used herein refers to a human female who is suffering from hot flashes. These hot flashes are typically associated with the natural onset of menopause (and, thus, includes menopausal and post menopausal women) but can also occur in "non-natural" settings, *e.g.*, when an oophorectomy has been performed. A human female "undergoing raloxifene administration" includes females who are ingesting raloxifene.

The need for the inhibition of bone loss in the context of the present invention may arise locally in cases of bone fracture, defect, prosthesis implantation, and the like. Such need may also arise in cases of systemic bone disease, such as osteoporosis, osteoarthritis, Paget's disease, multiple myeloma and other forms of cancer, bone loss resulting from side effects of other medical treatment (such as steroids), and age-related loss of bone mass.

Raloxifene may be made by established procedures, such as those detailed in U.S. Patent No.'s 4,418,068 and 5,629,425, the teachings of which are herein incorporated by reference. Fluoxetine, as a racemic mixture, may also be made by established procedures, such as those detailed in U.S. Patent No.'s 4,314,081 and 4,194,009, the teachings of which are herein incorporated by reference. Substantially pure, individual enantiomers of fluoxetine may be prepared by known procedures such as those taught in U.S. Patent No. 5,708,035, the teachings of which are herein incorporated by reference, and references cited therein. See also, Robertson, *et al., J. Med. Chem.,* 31:1412-1417, 1988 for a synthesis of (S)-fluoxetine.

Pharmaceutical formulations of the invention which include fluoxetine, or fluoxetine and raloxifene, for administration will generally include an effective amount of fluoxetine and an effective amount of raloxifene, when applicable, in addition to a pharmaceutically acceptable excipient. Formulations containing fluoxetine, without raloxifene, are taught in U.S. Patent No. 4,194,009, the teachings of which are herein incorporated by reference. Formulations containing raloxifene, without fluoxetine, are taught in U.S. Patent No. 4,418,068 and European Patent Application 95/301291.1, the teachings of which are herein incorporated by reference.

Suitable excipients include most carriers approved for parenteral administration, including water, saline, Ringer's solution, Hank's solution, and solutions of glucose, lactose dextrose, ethanol, glycerol, albumin, and the like. These compositions may optionally include stabilizers, antioxidants, antimicrobials, preservatives, buffering agents, surfactants, and other accessory additives. Fluoxetine, or fluoxetine and raloxifene, may also be delivered in an iontophoretic patch. A thorough discussion of suitable vehicles for parenteral administration may be found in E.W. Martin, "Remington's Pharmaceutical Sciences" (Mack Pub. Co., current edition sections relating to the excipient vehicles and formulating being incorporated herein by reference to disclose such). Such formulations are generally known to those skilled in the art and are administered systemically to provide systemic treatment.

If a combination of fluoxetine and raloxifene is administered as a single composition, the molar ratio of raloxifene to fluoxetine will be about 10:1 to 1:10, preferably about 4:1 to 1:3, and most preferably about 2.1:1 to 1.9:1.

The following formulation examples are illustrative only and are not intended to limit the scope of the present invention. The term "active ingredients" refers to fluoxetine and raloxifene.

Hard gelatin capsules are prepared using the following:

| Formulation 1 | |
|---|---|
| Gelatin Capsules | |
| Ingredient | Quantity (mg/capsule) |
| Fluoxetine | 0.1 - 1000 |
| Raloxifene | 0.1 - 1000 |
| Starch, NF | 0 - 650 |
| Starch flowable powder | 0 - 650 |
| Silicone fluid 350 centistokes | 0 - 15 |

The formulation above may be changed in compliance with the reasonable variations provided.

A tablet formulation is prepared using the ingredients below:

| Formulation 2 | |
|---|---|
| Tablets | |
| Ingredient | Quantity (mg/tablet) |
| Fluoxetine | 2.5 - 1000 |
| Raloxifene | 2.5 - 1000 |
| Cellulose, microcrystalline | 200 - 650 |
| Silicon dioxide, fumed | 10 - 650 |
| Stearate acid | 5 - 15 |

The components are blended and compressed to form tablets.

Alternatively, tablets each containing 2.5 - 1000 mg of each of the active ingredients are made up as follows:

| Formulation 3 | |
|---|---|
| Tablets | |
| Ingredient | Quantity (mg/tablet) |
| Fluoxetine | 25 - 1000 |
| Raloxifene | 25 - 1000 |
| Starch | 45 |
| Cellulose, microcrystalline | 35 |
| Polyvinylpyrrolidone | 4 |
| (as 10% solution in water) | |
| Sodium carboxymethyl cellulose | 4.5 |
| Magnesium stearate | 0.5 |
| Talc | 1 |

The active ingredients, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C-60°C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.1 - 1000 mg of each of the active ingredients per 5 ml dose are made as follows:

| Formulation 4 | |
|---|---|
| Suspensions | |
| Ingredient | Quantity (mg/5 ml) |
| Fluoxetine | 0.1 - 1000 mg |
| Raloxifene | 0.1 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The active ingredients are passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

An aerosol solution is prepared containing the following ingredients:

| Formulation 5 | |
|---|---|
| Aerosol | |
| Ingredient | Quantity (% by weight) |
| Fluoxetine | 0.25 |
| Raloxifene | 0.25 |
| Ethanol | 25.75 |
| Propellant 22 (Chlorodifluoromethane) | 70.00 |

The active ingredients are mixed with ethanol and the mixture added to a portion of the propellant 22, cooled to 30° C, and transferred to a filling device. The required amount is then fed to a stainless steel container and diluted with the remaining propellant. The valve units are then fitted to the container.

Suppositories are prepared as follows:

| Formulation 6 | |
|---|---|
| Suppositories | |
| Ingredient | Quantity (mg/suppository) |
| Fluoxetine | 250 |
| Raloxifene | 250 |
| Saturated fatty acid | 2,000 |
| glycerides | |

The active ingredients are passed through a No. 60 mesh U.S. sieve and suspended in the saturated fatty acid glycerides previously melted using the minimal necessary heat. The mixture is then poured into a suppository mold of nominal 2 g capacity and allowed to cool.

An intravenous formulation is prepared as follows:

| Formulation 7 | |
|---|---|
| Intravenous Solution | |
| Ingredient | Quantity |
| Fluoxetine | 50 mg |
| Raloxifene | 50 mg |
| Isotonic saline | 1,000 mL |

The solution of the above ingredients is intravenously administered to a patient at a rate of about 1 mL per minute.

When fluoxetine and raloxifene are both employed, they may be administered sequentially, concurrently, or simultaneously as a single composition to the subject. If administered sequentially, the period between the administration of fluoxetine and raloxifene will typically be one week to one month, and optimally, one day to one week. In a preferred administration scheme, the human female will receive fluoxetine and raloxifene concurrently or simultaneously.

In accordance with one method of use, fluoxetine and raloxifene may be administered systemically orally and/or parenterally, including subcutaneous or intravenous injection, and/or intranasally.

The precise dosage necessary will vary with the age, size, sex and condition of the subject, the nature and severity of the disorder to be treated, and the like; thus, a precise effective amount should be determined by the caregiver. In general terms, an effective dose of fluoxetine will range from about 0.001 mg/kg to about 5 mg/kg of body weight, per day. An effective dose for raloxifene is about 0.001 mg/kg to 10 mg/kg of body weight, per day. The total dosage (per day) of fluoxetine will typically be in the range from about 1 mg to 200 mg per day, usually being in the range from about 5 mg to 80 mg per day, preferably being in the range from about 10 mg to 60 mg per day, and more preferably being in the range from about 15 mg to 40 mg per day.

When fluoxetine is administered with raloxifene, the total dosage (per day) of raloxifene will typically be in the range from about 1 mg to 1000 mg per day, usually being in the range from about 10 mg to 100 mg per day, preferably being in the range from about 25 mg to 75 mg per day, more preferably being in the range from about 55 mg to 65 mg per day, and most preferably being 60 mg per day.

The following description is put forth so as to provide those of ordinary skill in the art with a complete disclosure and description of the effectiveness of the compositions and methods of the invention and are not intended to limit the scope of what the inventors regard as their invention.

A total of 120 - 160 women are gathered for a clinical trial. These women are either 1) naturally menopausal; or 2) pre-menopausal but had undergone bilateral oophorectomy surgery within four weeks prior to the commencement of the study. All the women in the study experience a minimum of thirty five hot flashes per week. The women are divided into four groups for a randomized double-blind placebo controlled study. The groups receive drug or placebo as illustrated below:
- Group 1:: Fluoxetine (20mg racemic mixture QD) + Placebo
- Group 2:: Placebo + Placebo
- Group 3:: Raloxifene (60mg QD) + Placebo
- Group 4:: Raloxifene (60mg QD) + Fluoxetine (20mg racemic mixture QD)

For three weeks all four groups are administered placebo only. For eight to twelve weeks thereafter, each group is administered drug, placebo, or some combination as outlined above. Data is collected (numbers/severity of hot flashes experienced) from each participant during and at the end of the test period.

The treatment of the clinical trial participants with fluoxetine results in a decrease, relative to placebo groups (Groups 2 and 3), of the incidence of hot flashes in the fluoxetine only group (Group 1) and fluoxetine/raloxifene group (Group 4). This decrease indicates the utility of the invention.

## Claims

1. A use of fluoxetine for the manufacture of a medicament for decreasing hot flashes in a human female.

2. The use according to Claim 1 where the fluoxetine is a racemic mixture of fluoxetine hydrochloride.

3. The use according to Claim 1 where the fluoxetine is substantially pure (R)-fluoxetine hydrochloride.

4. The use according to either Claim 2 or Claim 3 where the human female is menopausal.

5. The use according to either Claim 2 or Claim 3 where the human female is post-menopausal.

6. A use of fluoxetine for the manufacture of a medicament for decreasing hot flashes in a human female undergoing raloxifene administration.

7. The use according to Claim 6 where the fluoxetine is a racemic mixture of fluoxetine hydrochloride and the raloxifene is raloxifene hydrochloride.

8. The use according to Claim 6 where the fluoxetine is substantially pure (R)-fluoxetine hydrochloride and the raloxifene is raloxifene hydrochloride.

9. The use according to either Claim 7 or Claim 8 where the human female is menopausal.

10. The use according to either Claim 7 or Claim 8 where the human female is post-menopausal.

11. The use according to Claim 6 where the raloxifene administration arises from the existence of osteoporosis, or probable onset of osteoporosis.

12. A pharmaceutical formulation comprising fluoxetine and raloxifene in a pharmaceutically acceptable excipient.

13. The formulation according to Claim 12 where the fluoxetine is a racemic mixture of fluoxetine hydrochloride and the raloxifene is raloxifene hydrochloride.

14. The formulation according to Claim 12 where the fluoxetine is substantially pure (R)-fluoxetine hydrochloride and the raloxifene is raloxifene hydrochloride.

15. The formulation according to either Claim 13 or Claim 14 where the molar ratio of raloxifene to fluoxetine is 3:1 to 1:2.

16. The formulation according to either Claim 13 or Claim 14 where the molar ratio of raloxifene to fluoxetine is 2.1:1 to 1.9:1.
